# EUROPEAN PATENT APPLICATION

(11) **EP 2 891 999 A2**
(43) Date of publication of application: **08.07.2015**
(21) Application number: 14197217.4
(22) Date of filing: 10.12.2014
(51) Int. Cl.: G06F 19/00

(54) **Cloud systems for providing health-related services in a communication network and methods thereof**

(30) Priority: 11.12.2013 US 201361914625 P
(71) Applicant: H2 Inc., 1-1002 Georg Town, Grand Cayman (KY)
(72) Inventor: Chung, Wen-Chun, 114 Taipei (TW); Deng, Chu-Yie, 112 Taipei (TW)
(74) Representative: Chimini, Francesco

(57) **Abstract**

A cloud system for providing health-related services in a communication network includes an electronic device, at least one user device, at least one third-party device, and a cloud server. The electronic device performs a measurement to obtain health-related data. The user device sends or receives an invitation. The third-party device sends the invitation to or receives the invitation from the user device and establishes a companion relationship with the user device in response to the invitation. The cloud server provides a platform to the third-party device for interacting with the user device therewith, wherein the user device transmits or uploads the health-related data obtained from the electronic device or manually recorded by the user to the cloud server via the communication network to interact with the third-party device, such that the third-party device retrieves or receives the transmitted or uploaded data from the cloud server through the platform and performs a respective health-related operation based on the retrieved or received data.

## Description

### BACKGROUND OF THE INVENTION

### Field of the Invention

The disclosure relates generally to cloud systems and methods for providing health-related services in a communication network and related methods, and, more particularly, to cloud systems and methods for providing health-related services in a communication network.

### Description of the Related Art

Electronic devices such as medical devices and health-related monitoring devices with sensors and/or detectors (e.g. a pulse oximeter, a ventilator, an EKG device, or other health-related monitoring devices) are widely used for collecting or measuring medical and health-index data such as Blood Glucose level, Blood Pressure, EKG, Cholesterol, Uric Acid and other vital information. It is well known how to treat a medical disorder or to diagnose, treat or monitor the condition of a patient using those medical devices. Generally, the patient has to manually and periodically collect or measure medical and health-index data using the medical devices and brings the collected or measured medical and health-index data to the doctor for diagnosis. The doctor may then determine subsequent treatment for the patient based on the collected or measured medical and health-index data provided by the patient. However, the patient may only visit the doctor every month or longer, causing the doctor to be unable to monitor the patient's status between two visits and provide suitable treatment to the patient.

Therefore, the need exists for a system and method for monitoring the patient's status in real time and providing suitable treatment to the patient.

### BRIEF SUMMARY OF THE INVENTION

Cloud systems for providing health-related services in a communication network and related methods are provided.

An embodiment of a cloud system for providing health-related services in a communication network includes at least one electronic device, at least one user device, at least one third-party device, and a cloud server. The electronic device performs a measurement to obtain health-related data. The user device is to store the health-related data collected by the electronic device, and sends or receives an invitation. The third-party device sends/receives the invitation to/from the user device and establishes a companion relationship with the user device in response to the invitation. The cloud server which is coupled to the user device and the third-party device via the communications network provides a platform to the third-party device for interacting with the user device, wherein the user device transmits or uploads the health-related data obtained from the electronic device to the cloud server via the communication network to interact with the third-party device, such that the third-party device retrieves or receives the transmitted or uploaded data from the cloud server through the platform and performs a respective health-related operation based on the retrieved or received data.

In another embodiment, a method for providing health-related services in a communication network for use in a system is provided, wherein the system comprises at least one user device, at least one electronic device, a cloud server and at least one third-party device, and the cloud server is coupled to the user device and the third-party device via the communication network. The method comprises the steps of sending an invitation from the user device to the third-party device or from a third-party device to the user device for establishing a companion relationship therebetween, the cloud server providing a platform to the third-party device for interacting with the user device, and the user device transmitting or uploading the health-related data obtained from the electronic device to the cloud server via the communication network to interact with the third-party device, such that the third-party device retrieves or receives the transmitted or uploaded data from the cloud server through the platform and performs a respective health-related operation based on the retrieved or received data.

Other aspects and features of the present invention will become apparent to those with ordinary skill in the art upon review of the following descriptions of specific embodiments of the cloud systems for providing health-related services in a communication network and related methods.

### BRIEF DESCRIPTION OF THE DRAWINGS

The invention will become more fully understood by referring to the following detailed description with reference to the accompanying drawings, wherein:
Fig. 1 is a schematic diagram illustrating an embodiment of a cloud system for providing health-related services in a communication network of the invention;
Fig. 2 is a schematic diagram illustrating an embodiment of a user device of the invention; and
Fig. 3 is a flowchart of an embodiment of a method for providing health-related services in a communication network of the invention.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

Embodiments of the present disclosure disclose cloud systems for providing health-related services in a communication network, and related methods thereof for providing health-related services in a communication network, which can provide an invitation mechanism to create companion relationships between users (e.g. patients) and different third parties, including but not limited to caregivers, care providers, health insurance companies, drug stores, pharmacies, and other third parties, and provide a platform on the cloud to bridge the users and the third parties, so as to monitor the user's status in real time and provide suitable treatment or take corresponding action to the users based on the monitored data of the users.

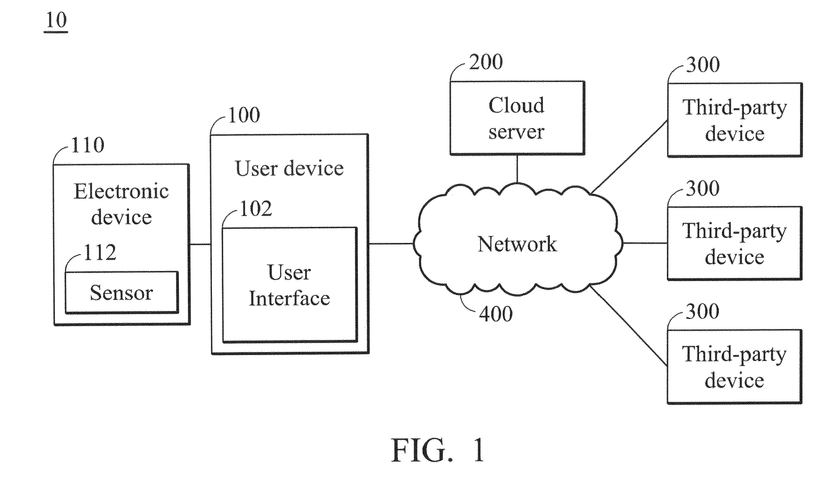

Fig. 1 is a block diagram of a cloud system for providing health-related services in accordance with an exemplary embodiment of the invention. As shown in Fig. 1, the cloud system 10 may comprise multiple user devices 100, multiple third-party devices 300, and a cloud server 200, wherein the user devices 100 are capable of communicating with the third-party devices 300 through the cloud server 200. To be more specific, the user devices 100 and the third-party devices 300 may be coupled to the cloud server 200 via a connected communication network 400 (e.g., any wired/wireless communication networks, such as the Internet, 3G, and/or WLAN network, etc...) and the user devices 100 and the third-party devices 300 may further communicate with the cloud server 200 via the connected communication network 400.

The user device 100 may include or be coupled to one or more electronic device 110 which comprises at least one or more sensors/detectors 112, wherein the sensors/detectors 112 are used for collecting/measuring various sensor data, such as various health-related vital data.

The electronic device 110 may perform a measurement to obtain health-related data. The electronic device 110 can be any electronic device which can collect monitored/sensor data (e.g. health-related data) via its sensor 112, such as medical devices and health-related monitoring devices with sensors and detectors to collect/measure medical and health indexes such as Blood Glucose level, Blood Pressure, EKG, Cholesterol, Uric Acid and so on. The medical device can be, but is not limited to, blood glucose meters, blood pressure meters, pulse oximeters, ventilators, EKG devices, thermometers, and various other vital data monitoring devices. The health-related monitoring devices can be, but is not limited to, pedometers, movement monitoring devices, sleep monitoring devices, and various other health-related monitoring devices. The user device 100 may provide user (e.g. a patient) information to the cloud server 200. For example, in one embodiment, the user of the user device 100 can be a patient and the user information can comprise patient-related data, which may comprise personal information about the patient (such as name, gender, telephone number, date of birth, or e-mail of the patient), the patient's vital information (e.g. the measured Blood Glucose level, Blood Pressure, EKG, Cholesterol, Uric Acid and so on) and/or other health-related data, such as food intake records, exercise records, medication records and so on. The user device 100 may provide measured data obtained from the electronic device 110 (e.g. the medical device) or the health-related data recorded on the user device 100 as the user information to the cloud server 200 each time a new measurement is performed on the user of the user device 100 via the electronic device 110 or a new data is recorded by the user of the user device 100. For example, in one embodiment, the electronic device is a blood glucose meter and the user device 100 (e.g. a smart phone) may provide measured data (e.g. the Blood Glucose level or Blood Pressure of the patient) obtained from the blood glucose meter as the user information to the cloud server 200 when there is a new measurement performed on the user of the user device 100 and thus the cloud server 200 can have the most recently measured data about the user of the user device 100. In one embodiment, a user (e.g. a patient) may record health-related data (e.g. body weight or food intake) on the user device 100, the user device 100 may further provide the health-related data recorded by the user to the cloud server 200 when there is new data recorded. In some embodiments, the users of the user device 100 may also be, but are not limited to, doctors, people of caregivers, people of care providers, people from health insurance companies, people from drug stores, pharmacies, and other companies which can provide health-related services.

The user device 100 may further provide a user interface 102 (e.g. an application) to obtain the medical and health-index data (e.g. the Blood Glucose value) which is collected or measured by the electronic devices 110, and provide information or further operations corresponding to the monitored data which is collected by the electronic device 110 and transmitted to the user device 100. The user interface 102, such as an application which is implemented by software (a so-called App), can provide a rich, friendly, and informative user experience based on data collection and analysis. For example, the App can display/provide the following information or functionalities: enable users to manually enter health-related data, provide a logbook, provide periodic summaries during a predefined time period (e.g. 7, 14, 30 day, etc.), identify high and low trends, provide graphs and charts of vital data for easy analysis for users or care providers, provide average readings of vital data, provide built-in reminders for users, send real-time alerts or analytics result to users, family members or care providers when triggered by pre-defined settings, provide data (e.g., logbook, graphs, or charts) extraction and share with family members or care providers, periodically or on an as-needed basis, and provide support for multiple users (by creating multiple accounts). In some embodiments, the App is capable of capturing, displaying and providing multiple vital signs (e.g. blood glucose + blood pressure) with the functionalities described in the section herein.

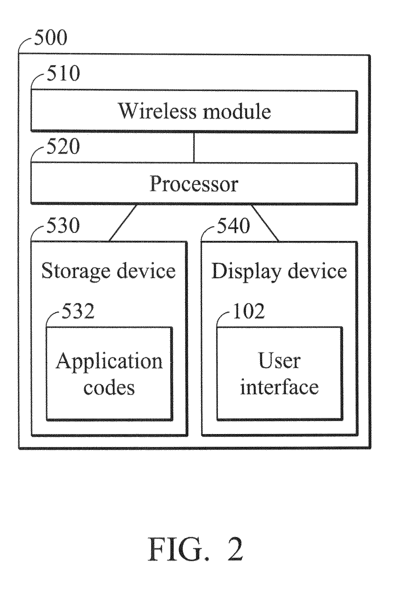

In some embodiments, the user device 100 can be a mobile device, such as a PDA, a smart phone, a mobile phone, a tablet, an MID, a laptop computer, a car computer, a digital camera, a multimedia player or a game device, or any other type of mobile computational device, however, it is to be understood that the invention is not limited thereto. Fig. 2 is a schematic diagram illustrating an embodiment of a user device of the invention. In this embodiment, the user device 500 is a mobile device and the mobile device may further comprise a wireless module 510, a processor 520, a storage device 530 and a display device 540. The wireless module 510 receives signals from and transmits signals to a current associated network. It is to be understood that integrating the processor 520 into the wireless module 510 is also possible. The wireless module 510 may be coupled to one or more antennas (not shown) and may allow communications with one or more additional devices, computers and/or servers using a wireless network. The mobile device may support various communications protocols, such as the code division multiple access (CDMA), Global System for Mobile Communications (GSM), Enhanced Data GSM Environment (EDGE), High-Speed Downlink Packet Access (HSDPA), Wi-Fi (such as IEEE 802.11a/b/g/n), Bluetooth, and Wi-MAX communication protocol, and a protocol for emails, instant messaging (IM), and/or a short message services (SMS), but the invention is not limited thereto. The processor 520 may be one or more data processors, image processors and/or central processors, which are capable of executing one or more types of computer readable medium stored in the storage device 530 such as a memory. In some embodiments, the wireless module 510 may be a cellular modem that provides mobile communication functionality based on the capabilities of the underlying hardware.

The storage device 530 may be a memory of the mobile device and also may be an external storage card, such as a smart media (SM) card or secure digital (SD) card, for example. The application codes 532 stored in the storage device 530 are executed by the processor 520 to display the user interface 102 on the display device 540 (e.g. a touch panel) for the user to monitor his health status based on the data obtained from the electronic device 110. The display device 540 is configured to display related data, such as texts, figures, interfaces, and/or related information. It is understood that, in some embodiments, the display device may be integrated with a touch-sensitive device (not shown). The touch-sensitive device has a touch-sensitive surface comprising sensors in at least one dimension to detect contact and movement of at least one object (an input tool), such as a pen/stylus or a finger near or on the touch-sensitive surface. Users can input relevant commands or signals via the screen of the display device 540.

The processor 520 which is coupled to the wireless module 510, the storage device 530 and the display device 540 can control the wireless module 510, the storage device 530 and the display device 540 to perform the method for providing health-related services of the present invention. To be more specific, the user of the mobile device may monitor the data measured by the electronic device 110 via the user interface 102.

In some embodiments, the user device 100 further activates the application to provide information or perform further operations corresponding to the health-related data by at least one of the following information or operations: displaying the health-related data on a display unit of the user device 100; displaying periodic summaries related to the health-related data during a predefined time period; identifying high and low trends related to the health-related data; providing warning information or built-in reminders for a user when the health-related data is lower or higher than a predetermined target; displaying graphs and charts corresponding to the health-related data; providing average readings related to the health-related data; sending real-time alerts to the third-party device 100 when triggered by pre-defined settings; extracting the health-related data and sharing the extracted data with the third-party device 300; and capturing, displaying and providing multiple vital signs related to the health-related data.

The cloud server 200 may enable communication between the user device 100 and the third-party devices 300 (e.g. a care provider or hospital's server or a doctor's display device). The cloud server 200 can enable the user device and the third-party devices 300 to connect and to access a platform provided by the cloud server 200. For example, the user device 100 can upload measured data received from the electronic device 110 to the cloud server 200, and the cloud server 200 may then store and/or pass the uploaded data to a dedicated third party device 300 (e.g. a care provider or hospital's server or a doctor's display device) such that the user of the dedicated third-party device 300 can continually track/monitor user/patient activity and perform subsequent operations accordingly.

In some embodiments, the cloud server 200 may further provide a specific web application as the platform to provide a web service that allows the third-party device 300 (e.g. PC, smartphone) to access the data stored on the cloud server 200 (such as user information with personal health-related data) from a webpage corresponding to the web service so that the third-party device 300 may access the data stored on the cloud server 200 via the specific web application. Similarly, the specific web application may obtain the medical and health-index data, and utilize the medical and health-index data for performing a particular operation, such as displaying the medical and health-index data on the display unit of a PC or smart phone, displaying periodic summaries, providing warning information (e.g. a pop-up warning message, a warning message sent over SMS or e-mail, a warning sound and so on) for the users (e.g. patients, family members, or care providers) when the medical and health-index data is lower/higher than a predetermined value, or displaying graphs and charts corresponding to the medical and health-index data.

In some embodiments, as shown in the exemplary embodiment of Fig. 1, the user device 100 may further transmit/upload the obtained data to the cloud server 200 via the connected network 400 for further processing. In another embodiment, the cloud server 200 may enable communication between the user device 100 and third-party devices 300 by providing a set of Application Programming Interfaces (APIs) as the platform. The APIs of the cloud server 200 can enable the users of dedicated third-party devices 300 to connect and to access the cloud server 200's platform. Third parties can also build services on top of the platform of the cloud server 200. For example, the user device can upload measured data received from the electronic device (e.g. a medical device) to the cloud server 200, and the cloud server 200 may then store and/or pass the uploaded data to a dedicated third-party devices (e.g. a service provider, care provider or hospital's server) using the APIs of the cloud server 200 such that the third party can continually track/monitor user/patient activity and perform subsequent operations accordingly via their third-party devices.

In some embodiments, the cloud server 200 may further provide a companion system which is a platform on the cloud, but stemming out to the user device (e.g. smart phones or PCs), to bridge the user of the user device 100 (e.g. the patients) and different third parties for providing health-related services, such as real time online diagnosis or suggestions, which will be described further in the following paragraphs. The third parties may include, but are not limited to, caregivers, care providers, health insurance companies, drug stores, pharmacies, and other third parties.

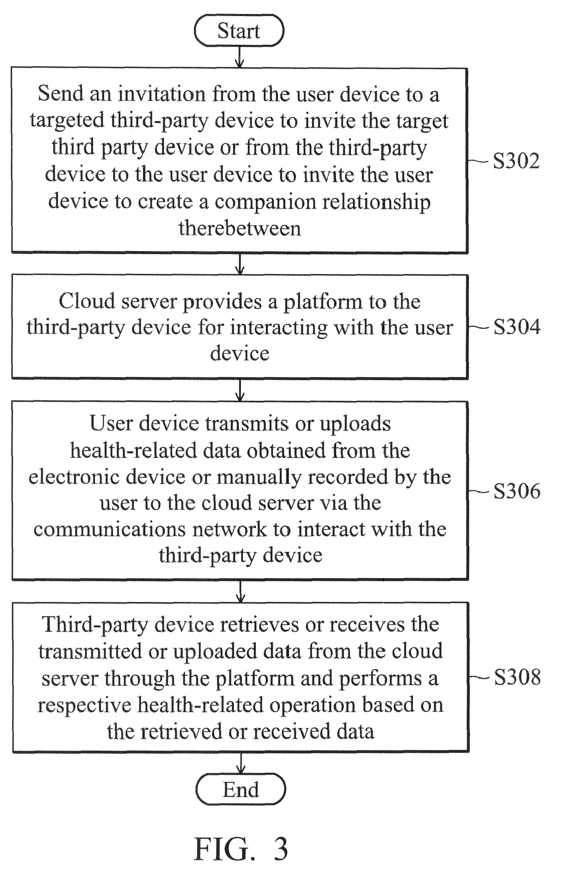

Fig. 3 is a flowchart of an embodiment of a method for providing health-related data in a communication network of the present disclosure.

First, in step S302, the user (e.g. a patient) of the user device 100 may want to allow a user (e.g. a doctor of the patient) of a targeted third-party device 300 to access and track health-related information from the cloud server 200, and thus an invitation is sent from the user device 100 to the targeted third-party device 300 to invite the targeted third-party device 300 to create a companion relationship and determine an access authority for the targeted third-party device 300. In some embodiments, a user (e.g. caregiver) of a targeted third-party device 300 may want to request to access and track health-related information of the user (e.g. a patient) of the user device 100 from the cloud server 200, so that the targeted third-party device 300 may send an invitation to the user device 100. The user device 100 may then accept the invitation and determine access authority for the targeted third-party device 300. In other embodiments, if the user of the targeted third-party device 300 is also a patient, the targeted third-party device 300 may invite the user device 100 to access and track health-related information of the user of the third-party device 300 or the user of the user device 100 may invite the user of the targeted third-party device 300 to request for access to the health-related information of the user of third-party device 300.

In some embodiments, the invitation is initialed by the user device 100. The user device 100 may send the invitation to the targeted third-party device 300, via SMS, Email or in-application message, for establishing the companion relationship between the user device 100 and the targeted third-party device 300. When the specific application has been installed on the user device 100 and the targeted third-party device 300, the user device 100 may initiate in-app invitation via the installed specific application by searching account name ("email"), for example. For example, the user device 100 may install a first application and send the invitation via an in-app message of the first application when the first application is installed on the third-party device. When it is not clear whether the targeted third-party device 300 has installed the specific application, the user device 100 may send the invitation to the third-party device 300 via, for example, an E-mail or Short Message Service (SMS) message. In one embodiment, the invitation may include information regarding a link to install the specific application, an invitation code to be input and a hash key will be further provided along with the invitation so that users can be notified and can install the specific application to accept the invitation and create the companion relationship.

For example, the user device 100 sends an invitation to the third-party device 300 and then the third-party device 300 receives the invitation within the first application installed or receives it in the form of email or SMS, provided with a temporary or permanent unique code generated by the cloud server 200. The third-party device 300 confirms the companion relationship with the user device 100 by accepting the invitation received within the first application, or to enter the unique code included in the received message into the first application.

In some embodiments, there may have two or more of the third-party devices 300, and the invitation may further comprise one-to-one invitation and one-to-many invitation, wherein the user device 100 may apply the one-to-one invitation to invite one of the third-party devices 300 to establish the companion relationship with it or apply the one-to-many invitation to invite a number of the third-party devices 300 (e.g. two or more of the third-party devices) to establish the companion relationship with each other using the temporary or permanent unique code assigned.

The user device 100 may determine the access authority of the invited targeted third-party device 300 when sending the invitation and the cloud server 200 may determine whether the third-party device 300 is allowed to access the health-related data of the user device 100 according to the access authority of the third-party device 300. In some embodiments, the access authority can be configured to a setting of limited access or a setting of full access. When the access authority of the invited targeted third-party device is configured for limited access, the user of the targeted third-party device 300 may send or receive messages to or from the user device 100 or schedule to send in-app reminders, and access to the personal health data are not allowed without proper authorization on behalf of the user. When the access authority of the targeted third-party device 300 is configured for full access, the user of the targeted third-party device 300 can not only send or receive messages to or from the user device 100 or schedule in-app reminders, but it can also access the personal health data of the user of the user device 100 from the cloud server 200, including diaries and graphs, or subscribe for alert notification when the user of the user device 100's vital data is beyond a predefined range, or when the user's vital data has not been uploaded to the cloud server 200 for a certain period of time. In some embodiments, the access authority is configured to a setting of full access by default, which means all of the users of the third-party devices 300 can not only send or receive messages to or from the user device 100 or schedule in-app reminders, but they can also access the personal health data of the user of the user device 100 from the cloud server 200.

Similarly, in some embodiments, the invitation is initialed by the third-party device 300. The targeted third-party device 300 may send the invitation to the user device 100, via SMS, Email or in-application message, for establishing the companion relationship between the user device 100 and the third-party device 300. When the specific application has been installed on the user device 100 and the third-party device 300, the third-party device 300 may initiate in-app invitation via the installed specific application by searching account name ("email"), for example. For example, the third-party device 300 may install a first application and send the invitation via an in-app message of the first application when the first application is installed on the user device 100. When it is not clear whether the user device 100 has installed the specific application, the third-party device 300 may send the invitation to the user device 100 via, for example, an E-mail or SMS message, provided with a temporary or permanent unique code assigned. In one embodiment, the invitation may include information regarding a link to install the specific application, an invitation code to be input and a hash key will be further provided along with the invitation so that users can be notified and can install the specific application to accept the invitation and confirm the companion relationship ..

For example, the third-party device 300 sends an invitation to the user device 100 and then the user device 100 receives the invitation within the first application installed or receives it in the form of email or SMS, provided with a temporary or permanent unique code generated by the cloud server 200. The user device 100 confirms the companion relationship with the third-party device 300 by accepting the invitation received within the first application, or to enter the unique code included in the received message into the first application.

In some embodiments, there may have two or more of the user devices 100, and the invitation may further comprise one-to-one invitation and one-to-many invitation, wherein the third-party device 300 may apply the one-to-one invitation to invite one of the user devices 100 to establish the companion relationship or apply the one-to-many invitation to invite a number of the user devices (e.g. two or more of the user devices) to establish the companion relationship using the temporary or permanent unique code assigned. For example, the user of a third-party device 300 (e.g. a doctor) may have the need to access to health-related data of users from multiple user devices 100, and the cloud server 200 may generate a temporary or permanent invitation code for the third-party devices 300. Thus, the third-party device 300 provides the invitation code to all of the user devices 100 installed with the first application, and each user device 100 can further input the invitation code into the first application to create the companion relationship with the third-party device 300. It should be understood that, in some embodiments, the invitation of creating the companion relationship may also be sent from one or more third-party devices 300 to other third-party devices 300 or from one or more user devices 100 to other user devices 100, and the invention is not limited thereto.

The third-party device 300 (e.g. another patient) may also determine the access authority of the invited user device 100 when sending the invitation and the cloud server 200 may determine whether a user device 100 is allowed to access the health-related data provided by the third-party device 300 according to the access authority of the user device 100. The configuration of access authority for the third-party device 300 is similar to the aforementioned configuration of access authority for the user device 100 and thus detail is not repeated here for brevity.

In step S304, the cloud server 200 provides a platform to the third-party device 300 for interacting with the user device 100. When the invitation is initialed by the user device 100, the targeted third-party device 300 may then receive the invitation from the user device 100 and establish a companion relationship with the user device 100 in response to the invitation. When the invitation is initialed by the third-party device 300, the user device 100 may then receive the invitation from the third-party device 300 and establish a companion relationship with the user device 100 in response to the invitation.

After the companion relationship between the user device 100 and the targeted third-party device 300 has been created, the targeted third-party device 300 may then access the cloud server 200 to obtain personal health information of the user of the user device 100. Thus, in step S306, the user device 100 transmits or uploads the health-related data obtained from the electronic device 110 or manually recorded by the user to the cloud server 200 via the communication network 400 to interact with the third-party device 300 and then, in step S308, the third-party device 300 retrieves or receives the transmitted or uploaded data from the cloud server 200 through the platform and performs a health-related operation based on the retrieved or received data. For example, the electronic device 110 can collect/measure medical and health-index data (such as Blood Glucose level or Blood Pressure value) using its sensor and the user device 100 acquires and receives the collected/measured medical and health-index data from the electronic device 110 and updates the collected or measured data as per the aforementioned user information to the cloud server 200 via the connected communication network 400. The cloud server 200 may store the uploaded data in a database. The targeted third-party device 300 may then access the cloud server 200 to obtain the user information including the medical and health-index data of the user of the user device 100.

A specific App on the user device 100 may be provided to detect and receive the collected or measured data to obtain the medical and health-index data (e.g. the Blood Glucose value), and utilize the medical and health-index data to perform a particular operation, such as displaying the medical and health-index data on the display unit of the user device 100, displaying periodic summaries, providing warning information (e.g. a pop-up warning message, a warning message sent by SMS, E-mail, or in-app message, a warning sound, and so on) to the user when the medical and health-index data is lower/higher than a predetermined target, or displaying graphs and charts corresponding to the medical and health-index data.

In one embodiment, the user device 100 may send and receive "in-app messages", via the specific application if the specific application has been installed on the user device, with any other third-party device 300 which has installed the specific application and created a companion relationship with the user device 100.

In some embodiments, the third-party devices 300 may be used by family and friends of the user, care providers (e.g. doctors, clinicians, certified nurses/educators and so on), payers (e.g. Insurance companies or employers) or other third parties (e.g. Pharmacies, retailers, manufacturers and so on), and the invention is not limited thereto. In one embodiment, for example, when a user provides a phone number under User Profile, and enables "auto match" function, the specific application can search for potential friends to find possible friends from the phonebook of the user.

After the companion relationship between the targeted third-party device 300 and the user device 100 has been created, the third-party device 300 may send and receive in-app messages (in the format of text, audio, or picture) to the user device 100 and perform relevant operations based on received or retrieved information. For example, the targeted third-party device 300 may access or retrieve updated data from the cloud server 200 and perform health-related services or operations based on received or retrieved information via the specific application.

The third-party device 300 may further perform the respective health-related operation based on the retrieved or received data by at least one of the following operations: performing a coaching operation to administer care and attention needed based on user-generated physiological data which is obtained from the retrieved or received data; performing a decision-making operation corresponding to the retrieved or received data; determining an inventory level of test trips based on the retrieved or received data; and sending electronic coupons or other sales promotion activities through the application.

In some embodiments, the third-party device 300 may also send in-app reminders to the user device 100. In another embodiment, the third-party device 300 may also access the personal health data of the user if it is authorized for full access.

In some embodiments, the third-party device 300 may further subscribe for alert notification to the cloud server 200 and the cloud server 200 further sends an alert message to the third-party device 300 when the health-related data indicates that any pre-defined setting has been matched. For example, the third-party device 300 may subscribe for alert notification when user's measured data (such as Blood Glucose value) is beyond a target range or the user's measured data has not been uploaded to the cloud server 200 for a certain period if authorized for Full access.

In some embodiments, the third-party device 300 may further perform a coaching operation to administer the required care and attention based on user-generated physiological data which may be uploaded from the user device 100.

In some embodiments, the third-party device 300 (e.g. the devices of physicians) may access measured data (such as Blood Glucose value) and other health-related data (such as food intake records, exercise records, medication records) belonging to the user of the user device 100 to support decision making, such as to administer changes in medication dosage, and the invention is not limited thereto.

In some embodiments, the user of the third-party device 300 (e.g. insurers or employers) may further track the wellness of users or employees via the platform or the specific application. In some embodiments, the user of the third-party device 300 (such as Pharmacies, retailers, or manufacturers) may further collect information on user behavior, determine the inventory level of test strips with each user since last purchase, and/or push electronic coupons or other sales promotion activities to users.

Therefore, the cloud systems for providing health-related services in a communication network and related methods thereof for providing health-related services in a communication network can provide a platform on the cloud to bridge the users and different third parties, so that the users can invite dedicated third parties with an access authority to selectively share with the health-related data and the user device can transmit or upload patient's health-related data to the cloud server via the connected network to interact with third parties to enabling the third parties to continually track or monitor user activity and perform subsequent operations accordingly, so as to monitor the users' status in real time and provide suitable treatment to the user based on the monitored data of the users or to send electronic coupons or other sales promotion activities to the users.

Methods, or certain aspects or portions thereof, may take the form of a program code (i.e., executable instructions) embodied in tangible media, such as floppy diskettes, CD-ROMS, hard drives, or any other machine-readable storage medium, wherein, when the program code is loaded into and executed by a machine, such as a computer, the machine thereby becomes an apparatus for practicing the methods. The methods may also be embodied in the form of a program code transmitted over some transmission medium, such as electrical wiring or cabling, through fiber optics, or via any other form of transmission, wherein, when the program code is received and loaded into and executed by a machine such as a computer, the machine becomes an apparatus for practicing the disclosed methods. When implemented on a general-purpose processor, the program code combines with the processor to provide a unique apparatus that operates analogously to application-specific logic circuits.

While the invention has been described by way of example and in terms of preferred embodiment, it should be understood that the invention is not limited thereto. Those who are skilled in this technology can still make various alterations and modifications without departing from the scope and spirit of this invention. Therefore, the scope of the present invention shall be defined and protected by the following claims and their equivalent.

## Claims

1. A cloud system for providing health-related services in a communication network, comprising:
an electronic device, performing a measurement to obtain health-related data;
at least one user device, sending or receiving an invitation;
at least one third-party device, sending the invitation to or receiving the invitation from the at least one user device and establishing a companion relationship with the at least one user device in response to the invitation; and
a cloud server coupled to the at least one user device and the at least one third-party device via the communication network, providing a platform to the at least one third-party device for interacting with the at least one user device therewith;
wherein the at least one user device transmits or uploads the health-related data obtained from the electronic device or manually recorded by the user of the user device to the cloud server via the communication network to interact with the at least one third-party device,
such that the at least one third-party device retrieves or receives the transmitted or uploaded data from the cloud server through the platform and performs a respective health-related operation based on the retrieved or received data.

2. The cloud system of claim 1, wherein the at least one user device further installs a first application and sends the invitation to the third-party device via an in-app message of the first application when the first application is installed on the at least one third-party device.

3. The cloud system of claim 1, wherein the at least one user device further sends the invitation to the at least one third-party device via an E-mail or Short Message Service (SMS) message, provided with a temporary or permanent unique code assigned.

4. The cloud system of claim 3, wherein the invitation further comprises a one-to-one invitation and one-to-many invitation, and the at least one user device further applies the one-to-one invitation to invite one of the at least one third-party device to establish the companion relationship or applies the one-to-many invitation to invite a number of the at least one third-party device to establish the companion relationship using the temporary or permanent unique code assigned.

5. The cloud system of claim 1, wherein the at least one third-party device further installs a first application and sends the invitation to the at least one user device via an in-app message of the first application when the first application is installed on the at least one user device.

6. The cloud system of claim 1, wherein the third-party device further sends the invitation to the at least one user device via an E-mail or Short Message Service (SMS) message, provided with a temporary or permanent unique code assigned.

7. The cloud system of claim 6, wherein the invitation further comprises a one-to-one invitation and one-to-many invitation, and the at least one third-party device further applies the one-to-one invitation to invite one of the at least one user device to establish the companion relationship or applies the one-to-many invitation to invite a number of the at least one user device to establish the companion relationship using the temporary or permanent unique code assigned.

8. The cloud system of claim 1, wherein the electronic device comprises a medical device and/or a health-related monitoring device with sensors and detectors to collect or measure medical and health-index data so as to generate the health-related data.

9. The cloud system of claim 1, wherein the at least one user device further activates an application to provide a user interface for recording the health-related data, obtaining the health-related data from the electronic device, and transmitting or uploading the health-related data to the cloud server, and providing information or performing further operations corresponding to the transmitted or uploaded data.

10. The cloud system of claim 1, wherein the platform is a web application and the at least one third-party device further retrieves or receives the transmitted or uploaded data from the cloud server and performs the respective health-related operation through the web application.

11. The cloud system of claim 1, wherein the platform is a set of Application Programming Interfaces (APIs) and the at least one third-party device further retrieves or receives the transmitted or uploaded data from the cloud server and performs the respective health-related operation through the set of APIs.

12. The cloud system of claim 1, wherein the at least one user device further provides access authority to the at least one third-party device when sending the invitation and the cloud server further determines whether the at least one third-party device is allowed to access the health-related data of the at least one user device according to the access authority of the at least one third-party device.

13. The cloud system of claim 1, wherein the user device further activates the application to provide information or perform further operations corresponding to the health-related data by at least one of the following information or operations:
displaying the health-related data on a display unit of the at least one user device;
displaying periodic summaries related to the health-related data during a predefined time period;
identifying high and low trends related to the health-related data;
providing warning information or built-in reminders for a user when the health-related data is lower or higher than a predetermined target;
displaying graphs and charts corresponding to the health-related data;
providing average readings related to the health-related data;
sending real-time alerts to the at least one third-party device when triggered by pre-defined settings;
extracting the health-related data and sharing the extracted data with the at least one third-party device; and
Supporting multiple users by creating multiple accounts and capturing, displaying and providing multiple vital signs related to the health-related data.

14. The cloud system of claim 1, wherein the at least one third-party device further subscribes to the cloud server for alert notification and the cloud server further sends an alert message to the at least one third-party device when the health-related data indicates that any pre-defined setting has been matched.

15. The cloud system of claim 1, wherein the at least one third-party device further performs the respective health-related operation based on the retrieved or received data by at least one of the following operations:
performing a coaching operation to administer care and attention needed based on user-generated physiological data which is obtained from the retrieved or received data;
performing a decision-making operation corresponding to the retrieved or received data;
determining an inventory level of test trips based on the retrieved or received data; and
sending electronic coupons or other sales promotion activities through the application.

16. A method for providing health-related services in a communication network for use in a system, wherein the system comprises at least one user device, an electronic device, a cloud server, and at least one third-party device, and the cloud server is coupled to the at least one user device and the at least one third-party device via the communication network, the method comprising:
sending an invitation from the at least one user device to the at least one third-party device to establish a companion relationship therebetween;
the cloud server providing a platform to the at least one third-party device for interacting with the at least one user device therewith; and
the at least one user device transmitting or uploading the health-related data obtained from the electronic device to the cloud server via the communication network to interact with the at least one third-party device,
such that the at least one third-party device retrieves or receives the transmitted or uploaded data from the cloud server through the platform and performs a respective health-related operation based on the retrieved or received data.
